# EUROPÄISCHE PATENTANMELDUNG

(11) **EP 1 967 148 A2**
(43) Veröffentlichungstag der Anmeldung: **10.09.2008**
(21) Anmeldenummer: 08002351.8
(22) Anmeldetag: 08.02.2008
(51) Int. Cl.: A61B 17/64

(54) **Vorrichtung zur vorübergehenden Fixierung von Gelenkteilen eines menschlichen Gelenks**

(30) Priorität: 08.03.2007 DE 102007011581
(71) Anmelder: Aequos Endoprothetik Gmbh, 82166 Gräfelfing (DE)
(72) Erfinder: Kubein-Meesenburg, Dietmar, 37070 Göttingen (DE); Nägerl, Hans, 37130 Gleichen (DE); Graf Stauffenberg, Caspar, 82166 Gräfelfing (DE)
(74) Vertreter: Scheffler, Jörg

(57) **Zusammenfassung**

Die Erfindung betrifft eine Vorrichtung (1) zur Festlegung eines distalen und eines proximalen Gelenkteils eines Gelenks mittels einander gegenüberliegend angeordneter Halteelemente (2), zwischen denen das jeweilige Gelenkteil mittels eines lösbaren Fixiermittels festlegbar ist. Die Vorrichtung (1) hat eine Führung (10) mit einer schlitzartigen Durchbrechung (11) für ein Operationsmittel, dessen Bewegungsfreiheitsgrad in sinnvoller Weise beschränkt ist, um den Operateur zu entlasten. Die Führung (10) ist zur bedarfsweisen Positionierung mit ihren Vorsprüngen (12) in eine von mehreren parallelen Nuten (13) einer Aufnahme der Vorrichtung (1) einschiebbar. Zur Auflage der beiden Gelenkteile in einer Flexionsstellung sind zwei konkave Auflagebereiche der Auflagefläche zueinander geneigt angeordnet. Zur einfacheren Handhabung der Vorrichtung (1) und zur Erleichterung der Operation bilden die Auflagefläche, die Halteelemente (2) sowie die Aufnahme für die Führung (10) eine gemeinsam an einer Grundplatte (16) der Vorrichtung (1) um eine Achse (17) schwenkbeweglich angeordnete Baueinheit.

## Beschreibung

Die Erfindung betrifft eine Vorrichtung zur vorübergehenden Fixierung von Gelenkteilen eines menschlichen Gelenks im Hand- und Fingerbereich mit zwei einander gegenüberliegend angeordneten Halteelementen, zwischen denen das jeweilige Gelenkteil mittels eines lösbaren Fixiermittels festlegbar ist.

Eine solche auch als Fixateur extern bezeichnete Vorrichtung dient als ein durch die Haut von außen befestigbares Haltesystem, um eine vorgegebene Gelenkstellung vorübergehend festzulegen, beispielsweise um einen Knochenbruch ruhigzustellen. Dabei wird die Fixierung auf beiden Seiten des Knochens verankert, der dann nach erfolgtem Einrichten des Knochens mit einer rigiden Vorrichtung, beispielsweise in Form von Stangen, in der vorbestimmten Position gehalten wird. Die Vorrichtung dient auf diese Weise dem Heilungsfortschritt.

Ein Fixateur extern wird beispielsweise bei offenen Frakturen mit Weichteilschädigungen, bei Trümmerfrakturen und gelegentlich Pseudarthrosen angewendet. Er kann auch verwendet werden, um Gelenke mit Absicht zu versteifen (sog. Arthrodese), so beispielsweise am Kniegelenk als gelenk-übergreifender Fixateur extern.

Durch die DE 198 56 890 A1 ist ein solcher Fingerfixateur bekannt, der die erforderlichen Bewegungsfreiheitsgrade eines Gelenks an einem gebrochenen Finger aufweist, welche insbesondere bei Trümmerfrakturen im Gelenk oder im gelenknahen Bereich zur Verhinderung einer Versteifung in den Gelenken erforderlich sind. Mit dem Fingerfixateur soll es möglich sein, eine Bewegung in definierter, vorgebbarer bzw. reproduzierbarer Form eines Gelenks an einem gebrochenen Finger ohne subjektiven Einfluss zu ermöglichen. Dafür werden zwei gelenkig miteinander verbundene stabförmige Elemente, an denen distale und proximale Knochenfixierelemente arretierbar sind, verwendet. Die distalen und proximalen Knochenfixierelemente werden beidseitig des Gelenks an einem gebrochenen Finger angeordnet. Die beiden stabförmigen Elemente sind mit mindestens einem mechanischen Antrieb verbunden, sodass das distale stabförmige Element um einen Drehpunkt oder eine Drehachse, der/die zumindest in der Nähe des Drehpunktes des Gelenks des Fingers liegt, schwenkbar ist.

Die DE 197 54 529 A1 betrifft einen Fingerfixateur zum Distrahieren von einzelnen Fingern entlang einer Kreisbahn, der über Knochenschrauben mit einem distalen und einem proximalen Fingerteil verbunden ist. Mit der Erfindung soll es möglich sein, einen verletzten Finger nicht nur zu verlängern, sondern auch gleichzeitig eine Formgebung des verletzten Fingers zu erreichen, wobei lediglich ein operativer Eingriff erforderlich ist und die Grundfunktion des Fingers durch die gleichmäßige Biegung wesentlich verbessert werden kann.

Die EP 06 85 206 A1 beschreibt ebenfalls einen externen Fixateur zur Behandlung von Frakturen im Hand- und Fingerbereich. Die hierbei verwendeten Drähte können übliche Kirschner-Drähte sein, die unmittelbar zwischen je zwei Muttern auf einer Gewindestange gehalten sind. Die hierzu bestimmten Queröffnungen sind als Längsschlitze ausgeführt, deren Dicke nicht wesentlich größer ist als die der Drähte.

Weitere Vorrichtungen zur Behandlung von Frakturen im Hand- und Fingerbereich sind durch die FR 2 855 040 A1, die JP 11290338 A, die DE 43 25 173 C1 sowie die US 6 063 087 A bekannt.

Der Erfindung liegt die Aufgabe zugrunde, eine Vorrichtung zur vorübergehenden Fixierung von Gelenkteilen eines menschlichen Gelenks im Hand- und Fingerbereich dahingehend zu verbessern, dass insbesondere eine operative Behandlung wesentlich vereinfacht wird.

Diese Aufgabe wird erfindungsgemäß mit einer Vorrichtung gemäß den Merkmalen des Anspruchs 1 gelöst. Die weitere Ausgestaltung der Erfindung ist den Unteransprüchen zu entnehmen.

Erfindungsgemäß hat also die Vorrichtung eine Auflagefläche für das distale Gelenkteil und das proximale Gelenkteil und eine Aufnahme für zumindest eine Führung, gegen welche ein Operationsmittel oder Operationshilfsmittel zur Beschränkung der relativen Beweglichkeit anlegbar ist. Hierdurch wird mit geringem Aufwand eine Möglichkeit zur einfachen und sicheren Behandlung geschaffen. Mit Hilfe der erfindungsgemäßen Vorrichtung ist eine zuverlässige und definierte Bearbeitung der Gelenkteile des Hand- und Fingerbereichs bei nahezu beliebigen Eingriffen, beispielsweise auch bei Operationsverfahren, möglich, bei denen ein oder mehrere Knochen gezielt durchtrennt und in der neuen Position fixiert werden. Eine ausschließlich dem Geschick des Operateurs überlassene Ausrichtung des Operationsmittels entfällt dabei. Vielmehr beschränkt die Führung der Vorrichtung die Bewegungsfreiheitsgrade des Operationsmittels relativ zu dem hierzu auf der sphärischen Auflagefläche festgelegten Gelenkteil in zweckmäßiger Weise, sodass die Handhabung für den Operateur wesentlich vereinfacht ist. Intraoperative Ausrichtzeiten werden durch Verlagerung in die präoperative Phase minimiert. Für komplexe Operationen wird die Möglichkeit geschaffen, einen Manipulator oder Roboter als operationsunterstützendes Operationshilfsmittel einsetzen zu können, sodass sich gegenüber den nach dem Stand der Technik bekannten Heilungshilfsmitteln ein neuartiges Anwendungsgebiet der so geschaffenen Vorrichtung erschließt.

Dabei erweist es sich als besonders vorteilhaft, wenn die Auflagefläche insbesondere mittels eines Schnellverschlusses lösbar mit der Vorrichtung verbunden ist, um so eine von der Fixierung unabhängige Positionierung zu ermöglichen. Hierzu wird das Gelenkteil zunächst auf der Auflagefläche fixiert, die dann ihrerseits an der Vorrichtung mittels des Schnellverschlusses positioniert wird. Der Schnellverschluss hat hierzu lediglich ein einziges Fixierelement, insbesondere ein Schnellspannelement, und ist mittels einer formschlüssigen Verbindung, insbesondere einer Rastverbindung, an der Vorrichtung festlegbar.

Eine besonders vorteilhafte Ausführungsform der erfindungsgemäßen Vorrichtung wird dadurch erreicht, dass die Führung eine schlitzartige Durchbrechung für das Operationsmittel, insbesondere eine oszillierende oder piezoelektrische Säge, oder Operationshilfsmittel, insbesondere Messmittel, aufweist. Hierdurch wird in einfacher Weise die gewünschte Bearbeitungsebene festgelegt, deren Winkelstellung gegenüber den Gelenkteilen mit reproduzierbarer Genauigkeit festgelegt ist.

Dabei erweist es sich als besonders praxisrelevant, wenn mittels der Führung das Operationsmittel oder das Operationshilfsmittel mit lediglich einem einzigen Freiheitsgrad beweglich führbar ist, um so beispielsweise eine axiale Schiebeführung zu realisieren, die dem Operateur eine wertvolle Unterstützung ermöglicht.

Die Aufnahme könnte beliebige an sich bekannte, beispielsweise auch kraftschlüssige Verbindungselemente aufweisen. Besonders zweckmäßig ist es hingegen, wenn die Aufnahme einen in eine Nut eingreifenden Vorsprung hat. Hierdurch ist nicht nur mit geringem Aufwand die relative Winkelstellung der Führung gegenüber den Gelenkteilen durch die Orientierung der Nut festgelegt, sondern durch eine Verschiebung des Vorsprungs in der Nut in Richtung des Gelenks kann zudem auch der Abstand variabel festgelegt werden.

Dabei erweist es sich als besonders praxisnah, wenn die Aufnahme eine Mehrzahl von bedarfsweise auswählbaren Nuten hat. Hierdurch wird eine besonders flexible Einsatzmöglichkeit der Vorrichtung zur Anpassung an unterschiedliche Gelenkteile oder zur individuellen Anpassung an den zu behandelnden Patienten geschaffen.

Eine andere, ebenfalls besonders zweckmäßige Ausgestaltung der erfindungsgemäßen Vorrichtung wird dadurch erreicht, dass die Vorrichtung mehrere Aufnahmen aufweist, von denen jeweils zumindest eine Auflagefläche dem distalen Gelenkteil und dem proximalen Gelenkteil zugeordnet ist, um so die Behandlung der beiden Gelenkteile ohne eine Änderung der Auflage durchführen zu können. Die Aufnahmen stimmen dabei beispielsweise hinsichtlich der Geometrie oder der Orientierung relativ zu der zugeordneten Auflagefläche überein und gestatten so die wahlweise Fixierung in derselben Führung.

Um zusätzlich auch eine beliebige Orientierung der Führung relativ zu dem jeweiligen Gelenkteil einstellen zu können, erweist es sich als vorteilhaft, wenn der Vorsprung an der Führung schwenkbeweglich angeordnet ist. Die Führung kann hierzu beispielsweise als ein im Wesentlichen zylindrisches Bauelement mit einer im Endbereich angeordneten, die Nut tragenden konzentrischen Lagerung ausgeführt sein. Die auf diese Weise realisierbare Drehachse verläuft dabei zweckmäßigerweise parallel zu der Schwenkachse des Gelenks.

Weiterhin wird eine besonders sinnvolle Ausgestaltung der erfindungsgemäßen Vorrichtung dadurch erreicht, dass die Führung in unterschiedlich beabstandeten Positionen zu dem Gelenk festlegbar ist, um so beispielsweise die Behandlungstiefe eines bestimmten Operationsmittels entsprechend festlegen zu können.

Eine besonders zuverlässige Fixierung der beiden Gelenkteile auf der jeweils zugeordneten Auflagefläche wird dadurch erreicht, dass das Halteelement ein in unterschiedlichen Positionen festlegbares Fixiermittel hat. Hierdurch kann der Operateur die Kontaktstelle der Fixiermittel an dem jeweiligen Gelenkteil individuell auswählen, um so die optimale Behandlung sicherzustellen.

Hierzu weist gemäß einer besonders einfachen Weiterbildung das Fixiermittel einen in eine Gewindeaufnahme des Halteelementes einschraubbaren Gewindeabschnitt auf, sodass einander gegenüberliegende Fixiermittel durch Einschrauben in das Halteelement sich zwisehen das jeweilige Gelenkteil festlegen. Ein solches auch als Knochenschraube ausführbares Fixiermittel kann daher in eine Vielzahl von an dem Halteelement vorhandenen Gewindeaufnahmen bedarfsweise in unterschiedlicher Anzahl eingeschraubt werden.

Hierzu hat das Fixiermittel einen dem Gelenkteil zugeordneten konischen Abschnitt, der sich dem Gewindeabschnitt als gelenkseitiger Endabschnitt anschließt und somit eine zuverlässige Festlegung des Gelenkteils gestattet. Der konische Abschnitt kann hierzu auch um die Schraubendrehachse des Fixiermittels zu diesem relativ drehbeweglich angebracht sein, sodass eine Übertragung der Drehbewegung auf den konischen Abschnitt vermieden wird. Selbstverständlich kann der konische Abschnitt auch als ein separates Bauteil mit einer Gewindeaufnahme ausgeführt sein, welche einen sich an dem Halteelement abstützenden Gewindebolzen aufnimmt, sodass an dem Halteelement keine Gewindeaufnahme erforderlich ist.

Eine andere, ebenfalls besonders praxisrelevante Ausgestaltung der vorliegenden Erfindung wird dadurch erreicht, dass die Auflagefläche, die Halteelemente sowie die Aufnahme für die Führung als eine gemeinsam an einer Grundplatte der Vorrichtung um eine zu der Gelenkachse parallele Achse schwenkbeweglich angeordnete Baueinheit ausgeführt sind. Hierdurch wird einerseits eine sichere Auflage der Vorrichtung einschließlich des darin fixierten Gelenks sichergestellt, andererseits ermöglicht die schwenkbewegliche Anordnung eine optimale Ausrichtung des Gelenks zur nachfolgenden Behandlung.

Weiterhin erweist es sich als besonders sinnvoll, wenn zur Auflage der beiden Gelenkteile in einer Gelenkstellung zwischen der Flexionsstellung und der Extensionsstellung zwei Auflagebereiche der Auflagefläche zueinander geneigt angeordnet sind, sodass jedes Gelenkteil optimal gehalten wird. Die Auflagebereiche können relativ zueinander in unterschiedlichen Positionen, insbesondere verschiedenen Neigungswinkeln festlegbar und gemäß einer weiteren vorteilhaften Abwandlung durch einen sphärischen Bereich verbunden sein, welcher das Gelenk in vorbestimmter Weise stützt.

Weiterhin ist es besonders praxisnah, wenn die Vorrichtung mit Markierungen, insbesondere einer Skalierung zur Einstellung der Relativposition der Führung gegenüber dem Gelenk, ausgestattet ist, sodass reproduzierbare Ergebnisse ermöglicht werden.

Zudem erweist es sich als sinnvoll, wenn zumindest einzelne der relativ zueinander beweglichen Elemente der Führung oder des Fixiermittels bei der Durchführung der Operation zur Vermeidung einer unbeabsichtigten Verlagerung mittels eines Anschlages begrenzbar, insbesondere festlegbar, sind, um so eine unbeabsichtigte Abweichung von einem vorbestimmten Behandlungsraum zu vermeiden.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt jeweils in einer perspektivischen Darstellung in
- Fig.1: eine erfindungsgemäße Vorrichtung mit einer Führung;
- Fig.2: eine vergrößerte Detaildarstellung der in Figur 1 gezeigten Vorrichtung;
- Fig.3: zwei jeweils mit mehreren Fixiermitteln ausgestattete Halteelemente der Vorrichtung;
- Fig.4: eine weitere erfindungsgemäße Vorrichtung.

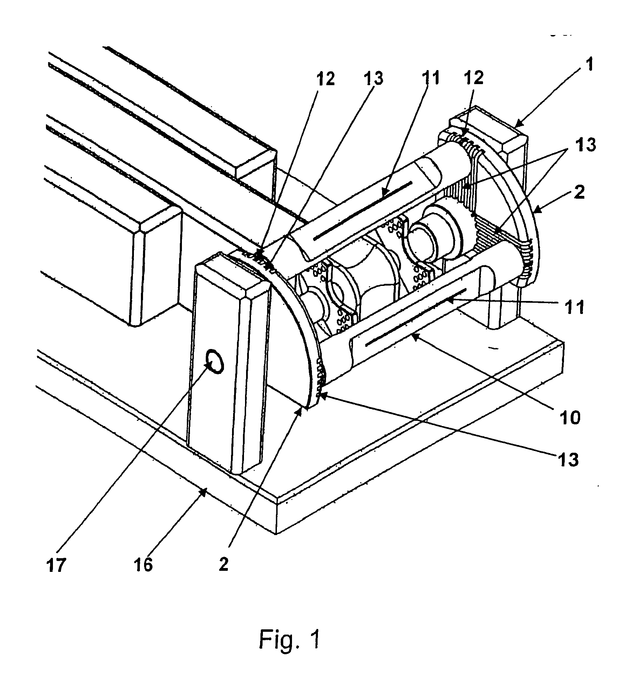

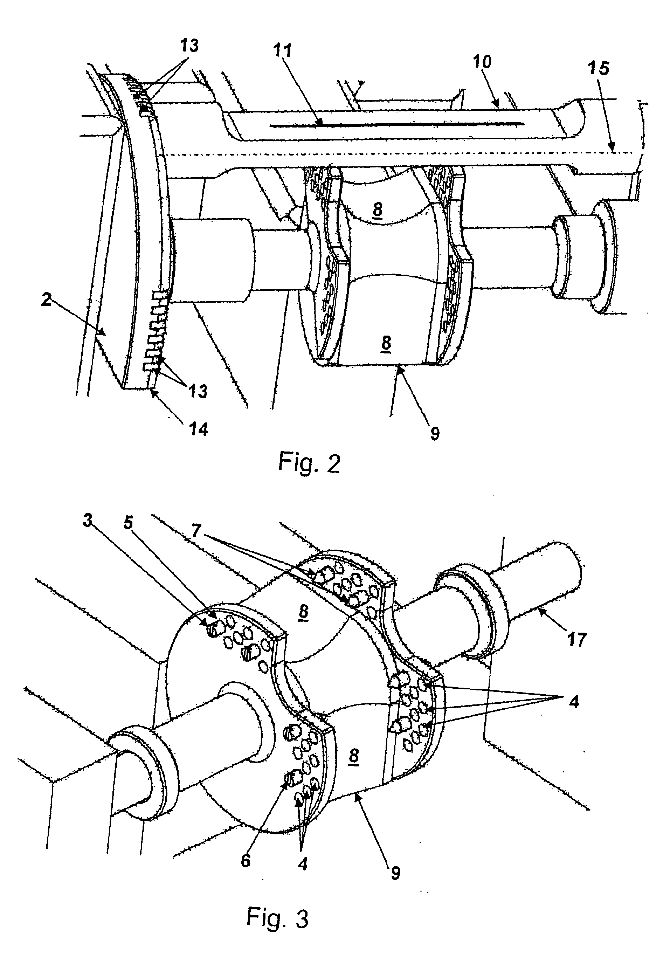

Eine erfindungsgemäße Vorrichtung 1 wird anhand der Figuren 1 bis 3 näher dargestellt, die jeweils eine perspektivische Darstellung der Vorrichtung 1 für ein nicht dargestelltes Gelenk zeigen. Die Vorrichtung 1 hat zur Festlegung eines distalen und eines proximalen Gelenkteils des Gelenks zwei einander gegenüberliegend angeordnete Halteelemente 2, zwischen denen das jeweilige Gelenkteil mittels eines lösbaren Fixiermittels 3 festlegbar ist. Hierzu hat das Fixiermittel 3 mehrere in eine jeweilige Gewindeaufnahme 4 des Halteelementes 2 mit einem Gewindeabschnitt 5 einschraubbare Knochenschrauben 6, die jeweils einen dem Gelenkteil zugeordneten konischen Abschnitt 7 aufweisen. Zur Auflage der beiden Gelenkteile in einer Flexionsstellung sind zwei konkave Auflagebereiche 8 der Auflagefläche 9 zueinander geneigt angeordnet. Die Vorrichtung 1 hat zusätzlich eine Führung 10 mit einer schlitzartigen Durchbrechung 11 für ein nicht dargestelltes Operationsmittel, dessen Bewegungsfreiheitsgrad dadurch in sinnvoller Weise beschränkt ist, um den Operateur zu entlasten. Die Führung 10 ist zur bedarfsweisen Positionierung mittels zweier Vorsprünge 12 in eine von mehreren parallelen Nuten 13 einer Aufnahme 14 der Vorrichtung 1 einschiebbar und durch axiales Spannen der Halteelemente 2 zwischen diesen festlegbar. Zugleich wird dadurch die Winkelstellung der relativ zu den Vorsprüngen 12 um ihre Mittelängsachse 15 schwenkbeweglichen Führung 10 festgelegt. Zur einfacheren Handhabung der Vorrichtung 1 und zur Erleichterung der Operation bilden die Auflagefläche 9, die Halteelemente 2 sowie die Aufnahme 14 für die Führung 10 eine gemeinsam an einer Grundplatte 16 der Vorrichtung 1 um eine Achse 17 schwenkbeweglich angeordnete Baueinheit.

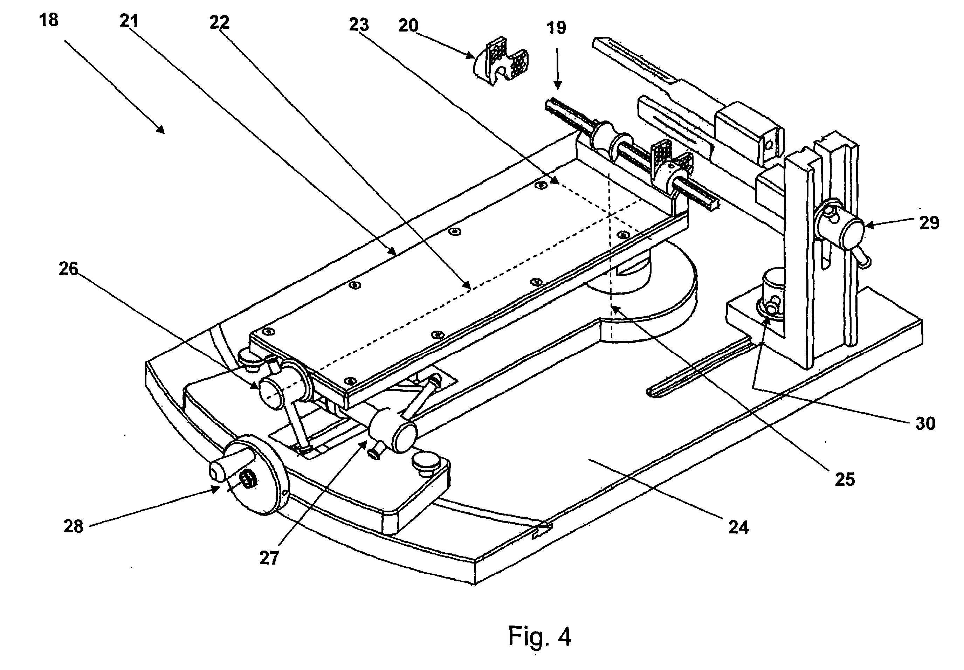

Eine weitere erfindungsgemäße Vorrichtung 18 ist in der Figur 4 in einer perspektivischen Darstellung gezeigt. Bei dieser Ausführungsform der Erfindung hat die Vorrichtung 18 zur Festlegung eines distalen und eines proximalen Gelenkteils des nicht gezeigten Gelenks zwei einander gegenüberliegend angeordnete und relativ auf ihrer gemeinsamen, als eine Keilwelle ausgeführten Achse 19 verschiebbare Halteelemente 20, zwischen denen das jeweilige Gelenkteil mittels eines lösbaren Fixiermittels festlegbar ist. Hierzu hat das Fixiermittel mehrere in eine Gewindeaufnahme 4 des jeweiligen Halteelementes 20 einschraubbare Knochenschrauben 6. Zur Auflage der beiden Gelenkteile in einer Flexionsstellung sind zwei konkave Auflagebereiche 8 der Auflagefläche 9 zueinander geneigt angeordnet und mit einer gemeinsamen Unterarmauflage 21 verbunden. Die Unterarmauflage 21 ist um zwei zueinander senkrechte Achsen 22, 23 in der Ebene der Unterarmauflage 21 sowie um eine gegenüber einer Grundplatte 24 der Vorrichtung 18 senkrechte Achse 25 schwenkbeweglich ausgeführt sowie jeweils mittels eines Stellmittels 26, 27, 28 in der gewünschten Position festlegbar. Die Vorrichtung 18 hat zusätzlich eine Führung 10 mit einer schlitzartigen Durchbrechung 11 für ein nicht dargestelltes Operationsmittel, dessen Position ebenfalls mittels eines Stellmittels 29 in ihrer gewünschten vertikalen Position sowie mittels eines weiteren Stellmittels 30 in verschiedenen Positionen an der Grundplatte 24 festlegbar ist.

## Patentansprüche

1. Instrumentelle Navigationshilfe für Gelenkoperationen mit einer Vorrichtung (1, 18) zur vorübergehenden Fixierung von Gelenkteilen eines menschlichen Gelenks insbesondere im Hand- und Fingerbereich mit zwei einander gegenüberliegend angeordneten Halteelementen (2, 20), zwischen denen das jeweilige Gelenkteil mittels eines lösbaren Fixiermittels (3) festlegbar ist, **gekennzeichnet durch** eine Auflagefläche (9) für das distale Gelenkteil und das proximale Gelenkteil und eine Aufnahme (14) für zumindest eine Führung (10), gegen welche ein Operationsmittel oder Operationshilfsmittel zur Beschränkung der relativen Beweglichkeit anlegbar ist.

2. Vorrichtung (1, 18) nach Anspruch 1, **dadurch gekennzeichnet, dass** die Auflagefläche (9) insbesondere mittels eines Schnellverschlusses lösbar mit der Vorrichtung verbunden ist.

3. Vorrichtung (1, 18) nach den Ansprüchen 1 oder 2, **dadurch gekennzeichnet, dass** der Schnellverschluss ein einziges Fixierelement, insbesondere eine Fixierschraube, hat.

4. Vorrichtung (1, 18) nach Anspruch 3, **dadurch gekennzeichnet, dass** der Schnellverschluss mittels einer formschlüssigen Verbindung, insbesondere einer Rastverbindung, an der Vorrichtung festlegbar ist.

5. Vorrichtung (1, 18) nach zumindest einem der vorhergehenden Ansprüche, **dadurch gekennzeichnet, dass** zumindest eines der Halteelemente (2, 20) zur Einstellung des Abstandes der Halteelemente (2, 20) in verschiedenen Positionen festlegbar ist.

6. Vorrichtung (1, 18) nach zumindest einem der vorhergehenden Ansprüche, **dadurch gekennzeichnet, dass** zumindest eines der Halteelemente (2, 20) entlang einer Achse (17, 19) verschiebbar und mittels einer kraftschlüssigen Verbindung festlegbar ist.

7. Vorrichtung (1, 18) nach zumindest einem der vorhergehenden Ansprüche, **dadurch gekennzeichnet, dass** die Halteelemente (2, 20) entlang der Achse (17, 19) gemeinsam verschiebbar angeordnet sind.

8. Vorrichtung (1, 18) nach zumindest einem der vorhergehenden Ansprüche, **dadurch gekennzeichnet, dass** die Führung (10) eine schlitzartige Durchbrechung (11) für das Operationsmittel, insbesondere eine oszillierende oder piezoelektrische Säge, oder ein Operationshilfsmittel, insbesondere ein Messmittel, aufweist.

9. Vorrichtung (1, 18) nach zumindest einem der vorhergehenden Ansprüche, **dadurch gekennzeichnet, dass** mittels der Führung (10) das Operationsmittel oder das Operationshilfsmittel mit lediglich einem einzigen Freiheitsgrad beweglich führbar ist.

10. Vorrichtung (1, 18) nach zumindest einem der vorhergehenden Ansprüche, **dadurch gekennzeichnet, dass** die Aufnahme (14) einen in eine Nut (13) eingreifenden Vorsprung (12) hat.

11. Vorrichtung (1, 18) nach Anspruch 10, **dadurch gekennzeichnet, dass** der Vorsprung (12) wahlweise in eine von mehreren Nuten (13) der Aufnahme (14) einführbar ist.

12. Vorrichtung (1, 18) nach Anspruch 10 oder 11, **dadurch gekennzeichnet, dass** der Vorsprung (12) an der Führung (10) schwenkbeweglich angeordnet ist.

13. Vorrichtung (1, 18) nach zumindest einem der vorhergehenden Ansprüche, **dadurch gekennzeichnet, dass** die Vorrichtung (1, 18) mehrere Aufnahmen (14) aufweist, von denen jeweils zumindest eine Auflagefläche (9) dem distalen Gelenkteil und dem proximalen Gelenkteil zugeordnet ist.

14. Vorrichtung (1, 18) nach zumindest einem der vorhergehenden Ansprüche, **dadurch gekennzeichnet, dass** die Führung (10) in unterschiedlich beabstandeten Positionen zu dem Gelenk festlegbar ist.

15. Vorrichtung (1, 18) nach zumindest einem der vorhergehenden Ansprüche, **dadurch gekennzeichnet, dass** die Führung (10) mittels einer Exzenterverstellung in unterschiedlichen Positionen in Bezug auf die Mittelängsachse (15) der Führung (10) festlegbar ist.

16. Vorrichtung (1, 18) nach zumindest einem der vorhergehenden Ansprüche, **dadurch gekennzeichnet, dass** zwei jeweils einem von zwei Gelenkkompartimenten desselben Gelenkteils zugeordneten Führungsbereiche der Führung unabhängig voneinander einstellbar ausgeführt sind.

17. Vorrichtung (1, 18) nach zumindest einem der vorhergehenden Ansprüche, **dadurch gekennzeichnet, dass** das Fixiermittel (3) an dem Halteelement (2, 20) in unterschiedlichen Positionen festlegbar ist.

18. Vorrichtung (1, 18) nach zumindest einem der vorhergehenden Ansprüche, **dadurch gekennzeichnet, dass** das Fixiermittel (3) einen in eine Gewindeaufnahme (4) des Halteelementes einschraubbaren Gewindeabschnitt aufweist.

19. Vorrichtung (1, 18) nach zumindest einem der vorhergehenden Ansprüche, **dadurch gekennzeichnet, dass** das Fixiermittel (3) einen dem Gelenkteil zugeordneten konischen Abschnitt (7) hat.

20. Vorrichtung (1, 18) nach zumindest einem der vorhergehenden Ansprüche, **dadurch gekennzeichnet, dass** die Auflagefläche (9), die Halteelemente (2, 20) sowie die Aufnahme (14) für die Führung (10) als eine gemeinsam an einer Grundplatte (16, 24) der Vorrichtung (1, 18) um eine zu der Gelenkachse parallele Achse (17, 22, 23) schwenkbeweglich angeordnete Baueinheit ausgeführt sind.

21. Vorrichtung (1, 18) nach zumindest einem der vorhergehenden Ansprüche, **dadurch gekennzeichnet, dass** zur Auflage der beiden Gelenkteile in einer Gelenkstellung zwischen der Flexionsstellung und der Extensionsstellung zwei Auflagebereiche (8) der Auflagefläche (9) zueinander geneigt angeordnet sind.

22. Vorrichtung (1, 18) nach Anspruch 21, **dadurch gekennzeichnet, dass** die Auflagebereiche (8) durch einen sphärischen Bereich verbunden sind.

23. Vorrichtung (1, 18) nach zumindest einem der vorhergehenden Ansprüche, **dadurch gekennzeichnet, dass** die Vorrichtung (1, 18) mit Markierungen, insbesondere einer Skalierung zur Einstellung der Relativposition der Führung (10) gegenüber dem Gelenk, ausgestattet ist.

24. Vorrichtung (1, 18) nach zumindest einem der vorhergehenden Ansprüche, **dadurch gekennzeichnet, dass** zumindest einzelne der relativ zueinander beweglichen Elemente der Führung (10) und/oder des Fixiermittels (3) zur Durchführung der Operation zur Vermeidung einer unbeabsichtigten Verlagerung mittels eines Anschlages begrenzbar, insbesondere festlegbar, sind.

25. Vorrichtung (1, 18) nach zumindest einem der vorhergehenden Ansprüche, **dadurch gekennzeichnet, dass** die Auflagefläche (9) mittels eines Gelenks, insbesondere mittels eines Kugelgelenks, lösbar mit der Vorrichtung verbunden ist.

26. Vorrichtung (1, 18) nach zumindest einem der vorhergehenden Ansprüche, **dadurch gekennzeichnet, dass** die Auflagefläche (9) mittels eines Gelenks um drei Achsen (22, 23, 25) unabhängig voneinander beweglich und in der gewünschten Position festlegbar ist.

27. Vorrichtung (18) nach zumindest einem der vorhergehenden Ansprüche, **dadurch gekennzeichnet, dass** die Auflagefläche (9) mit einer Unterarmauflage (21) verbunden ist.

28. Vorrichtung (18) nach Anspruch 27, **dadurch gekennzeichnet, dass** die Unterarmauflage (21) um zwei zueinander senkrechte Achsen (22, 23) in der Ebene der Unterarmauflage (21) sowie um eine gegenüber einer Grundplatte (24) der Vorrichtung (18) senkrechte Achse (25) schwenkbeweglich ausgeführt ist.

29. Vorrichtung (1, 18) nach zumindest einem der vorhergehenden Ansprüche, **dadurch gekennzeichnet, dass** die Vorrichtung (1, 18) mit einem Instrument zu Erfassung des relativen Versatzes mehrerer Gelenkkompartimente eines Gelenkteils, insbesondere Gelenkkopfes, ausgestattet ist.

30. Vorrichtung (1, 18) nach zumindest einem der vorhergehenden Ansprüche, **dadurch gekennzeichnet, dass** die Auflagefläche (9) und/oder die zumindest eine Führung (10) zur unabhängigen Behandlung einzelner Gelenkkompartimente ausgeführt ist.
